# EUROPEAN PATENT APPLICATION

(11) **EP 1 065 268 A2**
(43) Date of publication of application: **03.01.2001**
(21) Application number: 00113524.3
(22) Date of filing: 26.06.2000
(51) Int. Cl.: C12M 1/107, C12P 5/02

(54) **Method of producing a biogas from sugar beet remains and a device for carrying out the method**

(30) Priority: 28.06.1999 SK 88899
(71) Applicant: Hutnan, Miroslav, 84107 Bratislava (SK); Drtil, Miloslav, 82108 Bratislava (SK); Mico, Stefan, 91106 Trencin (SK); Valach, Jozef, 91108 Trencin (SK)
(72) Inventor: Hutnan, Miroslav, 84107 Bratislava (SK); Drtil, Miloslav, 82108 Bratislava (SK); Mico, Stefan, 91106 Trencin (SK); Valach, Jozef, 91108 Trencin (SK)
(74) Representative: Bergmeier, Werner, Dipl.-Ing.

(57) **Abstract**

Method for producing a biogas from sugar beet cuts, especially as the remains from beet sugar production, is based in that the sugar beet remains are mixed with water forming a substrate with true dry matter 1 to 10% by volume with postponing period from 1 to 6 days, which is hydrolyzed at the temperature 15 to 50°C with simultaneous acidification at pH 4.0 to 6.0 with subsequent effect of methanizing bacteria releasing biogas in continuous or discontinuous multistage process. Device for carrying out the method comprises hydrolyzation - acidification tank (1) preceded by preliminary tank (9).

## Description

### Field of the invention

The invention is concerning a method for producing a biogas from sugar beet cuts, especially as the remains from beet sugar production and device for carrying out the method.

### Background art

Processing of sugar beet remains from sugar production is covered mainly in the works of Lane, A. G., Biomass 5, 245-259, 1985 and Weiland P. Water Sci. Technol. 27, 145-151, 1993, that introduce sugar beet remains from sugar production as one of the convenient substrates for anaerobic processing in order to produce biogas.

Disadvantage of the methods mentioned in cited works lays in the fact, that anaerobic processing of sugar beet remains was held without pretreatment, or only liquid phase from pretreatment in hydrolyzation - acidification stage, was used. The significant disadvantage of such sugar beet remains processing is low capacity, that is only 4.06 kg.m⁻³ and low efficiency to chemical oxygen demand, only 60 to 65%.

The purpose of the invention is therefore to develop such method for obtaining a biogas from sugar beet remains, that the high energy utilization of biomass is achieved.

### Summary of the invention

Purpose of the invention is achieved by the method for producing a biogas from sugar beet remains, especially from beet sugar production, which bases in that the sugar beet remains are mixed with water forming a substrate with true dry matter 1 to 10% by volume that is hydrolyzed at the temperature 15 to 50°C with simultaneous acidification at pH 4.0 to 6.0 and with subsequent effect of methanizing bacteria releasing a biogas, in continuous or discontinuous multistage mode.

Device for carrying out the method comprises hydrolyzation - acidification tank, which is in upper part provided with dosing throat and in lower part provided with discharge piping. Hydrolyzation - acidification tank is preceded by preliminary tank, which is provided with water inlet pipe and the substrate outlet pipe and with filling hole in the upper part.

It is advantageous when hydrolyzation - acidification tank comprises a stirring device.

It is also advantage, when hydrolyzation - acidification tank has heating element with inlet and outlet of heating medium, located above the tank bottom.

Method according to the invention has high utilization characteristics, during the process that passes in regular physical conditions without the use of chemical agents. High capacity allows significant decrease in necessary volumes and high energy utilization of biomass.

### Description of the drawings

The invention is closely described in drawing, which schematically shows device for producing a biogas from sugar beet remains.

### Description of the embodiments

### Example 1

Sugar beet remains are mixed with water in discontinuous multistage mode forming a substrate which true dry matter is 1% by volume with 1 day postponing period. Substrate is then hydrolyzed at the temperature 20°C with simultaneous acidification at pH 4.5 and subsequent effect of methanizing bacteria that release biogas. Specific biogas production is 0.3 m³ of 1 kg sugar beet remains with 50% volume of methan.

### Example 2

Sugar beet remains are mixed with water in continuous multistage mode forming a substrate which true dry matter is 10% by volume with 6 days postponing period. Substrate is hydrolyzed at the temperature 50°C with simultaneous acidification at pH 6.0 and subsequent effect of methanizing bacteria that release biogas. Specific biogas production is 0.55 m³ of 1 kg sugar beet remains with 80% volume of methan.

Plant for producing a biogas from sugar beet remains comprises hydrolyzation - acidification tank **1** with circle or angular plan, which is in upper part provided with dosing throat **10** for dosing a substrate. Tank lower part is provided with substrate discharge piping **7**. Hydrolyzation - acidification tank is provided with stirring device **6** mounted on the top. Heating equipment **4** with inlet of heating medium **41** and outlet of cooled medium **42**, is situated above the tank bottom. Hydrolyzation - acidification tank is preceded by preliminary tank **9** provided with filling hole **2** in the upper part and comprises water inlet pipe **3** and substrate outlet pipe **8**.

Preliminary tank **9** is filled through filling hole **2** with sugar beet remains and water forming a substrate which is pumped through pipe **8** and dosing throat **10** into hydrolyzation - acidification tank **1**, in the manner that, true dry matter is 1 to 10% by volume and postponing period from 1 to 6 days, what corresponds with capacity 1 to 30 kg.m⁻³, where sugar beet remains are hydrolyzed and acidified at the temperature 15 to 50°C, and afterwards they are pumped to methanizing reactor (not represented in drawing), where a biogas is released by the effect of methanizing bacteria.

Device is of simple construction with minimum space requirements and with high efficiency in subsequent methanizing decomposition. It reliably covers economical demands on utilization of sugar beet remains for biogas production. Specific biogas production is 0.3 to 0.55 m³ of one kilogram sugar beet remains with 50 to 80% volume of methan.

### Industrial Applicability

Device for producing a biogas from sugar beet remains, especially from beet sugar production or in fabrics utilizing already built methanizing stage for obtaining a biogas. Biogas produced with method according to the invention can be used either for direct burning in water heating appliances or can be used along with other fuels for electricity and hot water production.

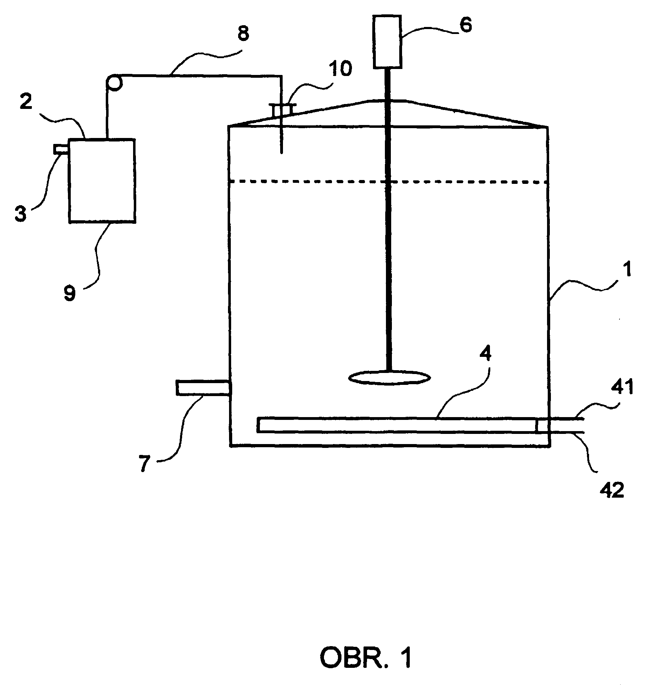

## Claims

1. Method for producing a biogas from sugar beet remains, especially from beet sugar production, **characterized in that**, the sugar beet remains are mixed with water forming a substrate with true dry matter 1 to 10 % by volume and with postponing period from 1 to 6 days, which is hydrolyzed at the temperature 15 to 50°C with simultaneous acidification at pH 4.0 to 6.0 and subsequent effect of methanizing bacteria releasing biogas, in continuous or discontinuous multistage mode.

2. Device for carrying out the method according to claim 1, comprising hydrolyzation - acidification tank (1), **characterized in that**, the hydrolyzation - acidification tank (1) provided with dosing throat 10 in upper part and with discharge piping (7) in lower part is preceded by preliminary tank (9) provided with water inlet pipe (3) and substrate outlet pipe (8) and comprises a filling hole (2) in its upper part.

3. Device according to claim 2, **characterized in that**, the hydrolyzation - acidification tank (1) is provided with stirring device (6).

4. Device according to claims 2 and 3 **characterized in that**, the hydrolyzation - acidification tank (1) comprises a heating equipment (4) with heating medium inlet (41) and cooled medium outlet (42), situated above the tank bottom.
